# FASCICULE DE BREVET EUROPEEN

(11) **EP 2 582 329 B1**
(45) Date de publication et mention de la délivrance du brevet: **25.02.2015**
(21) Numéro de dépôt: 11731117.5
(22) Date de dépôt: 17.06.2011
(51) Int. Cl.: A61F 2/34, A61F 2/46, A61F 2/00

(54) **PROCEDE DE FABRICATION D'UN COTYLE CERAMIQUE**
VERFAHREN ZUR HERSTELLUNG EINES GELENKPFANNE AUS KERAMIK
METHOD FOR PRODUCING A CERAMIC ACETABULUM

(30) Priorité: 17.06.2010 FR 1054834
(43) Date de publication de la demande: 24.04.2013
(73) Titulaire: Inventorio SA, 1196 Gland (CH)
(72) Inventeur: Gradel, Thomas, 74970 Marignier (FR)
(74) Mandataire: Poncet, Jean-François
(86) Numéro de dépôt international: PCT/IB2011/052649
(87) Numéro de publication internationale: WO 2011/158213

(56) Documents cités:
- EP-A1- 0 931 523
- EP-A1- 1 721 586
- EP-A1- 2 442 754
- EP-A2- 2 482 762
- WO-A1-2009/098491
- WO-A1-2010/146398
- DE-A1- 3 101 333
- DE-C1- 4 403 994
- FR-A1- 2 830 746
- FR-A1- 2 897 527
- FR-A1- 2 909 541
- FR-A1- 2 950 525
- US-A- 5 486 181
- US-A- 5 888 205
- US-B1- 6 682 567

## Description

### DOMAINE TECHNIQUE DE L'INVENTION

La présente invention concerne un procédé de fabrication d'un cotyle prothétique destiné à remplacer le cotyle naturel de la hanche.

Une prothèse totale de hanche comprend deux parties constituant une articulation à rotule, à savoir une partie femelle destinée à remplacer le cotyle naturel de la hanche et une partie mâle destinée à remplacer la tête du fémur. La partie mâle de l'articulation comporte généralement une tige destinée à pénétrer dans le canal médullaire du fémur, et dont l'extrémité proximale se raccorde par un col à une tête prothétique sphérique destinée à pénétrer dans la partie femelle. La partie femelle de l'articulation, qui doit remplacer le cotyle naturel de la hanche, comprend habituellement une cupule à face extérieure d'ancrage convexe qui vient se loger dans une cavité cotyloïdienne préparée de l'os du bassin. Ladite cupule comporte une face de réception concave destinée à recevoir, directement ou indirectement, la tête prothétique sphérique.

Lors de la pose de la cupule dans la cavité cotyloïdienne, il faut pouvoir utiliser un impacteur qui permet de tenir et manipuler la cupule et de lui appliquer une force d'enfoncement dans la cavité cotyloïdienne de l'os avec une bonne orientation, pendant une durée suffisante notamment pour la prise d'un ciment entre la surface externe de la cupule d'insertion et la cavité cotyloïdienne de l'os.

Lorsque la cupule est métallique, une telle matière permet aisément de prévoir des moyens de fixation fiable d'un impacteur. A cet effet, le document US 5,486,181 décrit la fixation d'un impacteur au moyen d'ailettes, tandis que le document US 5,888,205 décrit un trou de sommet fileté pratiqué dans la face de réception concave.

Dans le cas particulier d'une cupule intégralement en céramique, ces solutions se révèlent inapplicables. La céramique est en effet trop fragile pour que soient réalisées des ailettes ou un trou fileté. Un trou fileté n'est en outre pas acceptable car, dans le cas d'une cupule en céramique, la face de réception concave est destinée à recevoir directement la tête prothétique sphérique : la face de réception concave doit ainsi être impeccablement lisse et ne saurait donc comporter le moindre usinage.

Les documents EP 0 931 523 et FR 2 830 746 décrivent des impacteurs destinés à être fixés à une cupule par l'intermédiaire de doigts d'accrochage pivotants. Le document WO 2009/098491 décrit un impacteur destiné à être fixé à une cupule par l'intermédiaire de baïonnettes rotatives. Tous ces impacteurs sont complexes, onéreux et procurent une retenue peu efficace de la cupule.

Le document FR 2 909 541 décrit une cupule à face extérieure d'ancrage convexe et hémisphérique. Pour tenir cette cupule lors de son impaction, il est prévu une gorge annulaire externe au voisinage de son bord libre annulaire. Cette gorge annulaire permet la fixation d'un impacteur comportant un corps de base à au moins deux pattes élastiques en forme de crochet. Dans ce document, la force de retenue de la cupule par l'impacteur est en corrélation avec et est sensiblement égale à la force qu'il est nécessaire d'appliquer pour écarter les pattes élastiques. Ceci signifie que meilleure est la tenue de la cupule par l'impacteur plus l'écartement des pattes est difficile, ce qui oblige l'utilisateur à peiner d'autant plus pour fixer l'impacteur à la cupule. Cela augmente le risque d'endommagement accidentel de la cupule, ce qui est critique dans le cas d'une cupule en céramique qui est en matériau fragile.

Le document EP 1 721 586 décrit une cupule sur laquelle est fixé un insert de pose et d'orientation. La cupule comporte une face de réception interne délimitée par une lèvre périphérique dans l'épaisseur de laquelle sont prévus une pluralité de logements conformés pour recevoir et retenir des languettes portées par la face inférieure de l'insert de pose et d'orientation. La réalisation des logements sans fragiliser ni détériorer la cupule n'est pas toujours possible selon l'épaisseur de la lèvre périphérique et le matériau de la cupule. Et les languettes demeurent des appendices relativement fragiles, de sorte qu'une tenue fiable de la cupule n'est pas assurée.

Il y a un besoin de permettre une fixation aisée d'un impacteur à une cupule tout en procurant une tenue fiable de la cupule.

### EXPOSE DE L'INVENTION

Un problème proposé par l'invention est de proposer un procédé de fabrication d'un cotyle prothétique de hanche à cupule en céramique qui puisse être fixé de façon encore plus fiable et robuste à un impacteur, en limitant les efforts à accomplir et en évitant tout risque de contamination.

Pour atteindre ces buts ainsi que d'autres, l'invention propose un procédé de fabrication comprenant les étapes de :
a) prévoir une cupule en céramique ayant une face de réception interne concave et ayant une structure annulaire de réception externe,
b) prévoir un insert de pose et d'orientation à structure périphérique annulaire de fixation conformée pour coopérer avec la face de réception de la structure annulaire externe de réception en s'engageant autour de la structure annulaire externe de réception,
c) chauffer l'insert de pose et d'orientation afin d'en augmenter les dimensions,
d) engager la structure périphérique annulaire de fixation autour de la face de réception de la structure annulaire externe de réception,
e) ramener à température ambiante l'insert de pose et d'orientation pour en diminuer les dimensions de façon à obtenir un serrage radial de la structure périphérique annulaire de fixation sur la structure annulaire externe de réception.

Un tel procédé de fabrication permet d'obtenir un serrage radial satisfaisant de l'insert de pose et d'orientation sur la cupule en céramique, et une retenue efficace de l'insert de pose sur la cupule en céramique.

La retenue en force par un serrage radial de l'insert de pose et d'orientation sur la cupule d'insertion assure une fixation fiable et solide qui permet d'endurer les efforts subis lors de l'impaction de la cupule d'insertion. Le serrage radial de la structure périphérique annulaire de fixation sur la structure annulaire externe de réception permet d'assurer cette fiabilité de fixation sans compliquer outre mesure la forme de la structure annulaire externe de réception.

La retenue de l'insert de pose et d'orientation sur la cupule d'insertion est très fiable, notamment grâce au fait que le serrage radial se fait sur une structure annulaire de réception qui est externe et qui présente donc une grande surface de contact avec l'insert de pose et d'orientation.

Enfin, le serrage radial de la structure périphérique annulaire de fixation sur la structure annulaire externe de réception induit un état de précontrainte dans la cupule en céramique, et cette précontrainte limite les risques de bris de la cupule lors de son impaction dans le cotyle du bassin d'un patient mais également lors de son transport et de ses manipulations lorsque le procédé selon l'invention est mis en oeuvre en usine.

Lorsque l'insert de pose et d'orientation est monté en usine sur la cupule, le chirurgien n'a plus qu'à fixer l'impacteur sur l'insert de pose et d'orientation pour procéder à l'impaction de la cupule. Il n'y a pas de risque que le chirurgien vienne dégrader la face de réception interne concave de la cupule d'insertion, celle-ci étant protégée par l'insert de pose et d'orientation.

Il doit être souligné que le fait de prévoir une fixation de l'insert de pose et d'orientation à la cupule au moyen d'une dilatation par chauffage (puis d'un retour à température ambiante) de l'insert de pose et d'orientation est particulièrement avantageux par rapport à une première solution alternative qui consisterait à refroidir la cupule plutôt que l'insert de pose et d'orientation pour en diminuer les dimensions extérieures (suivi d'un retour à température ambiante) ou par rapport à une deuxième solution alternative qui consisterait à prévoir un engagement de l'insert de pose et d'orientation à l'intérieur de la cupule et à effectuer un refroidissement dudit insert de pose et d'orientation pour en diminuer les dimensions extérieures (suivi d'un retour à température ambiante).

En effet, ces solutions alternatives nécessitent une enceinte réfrigérée dans laquelle peut se produire un phénomène de condensation menant à la présence de liquides de condensats sur la cupule ou l'insert de pose et d'orientation, lesquels liquides peuvent être contaminés par des bactéries ou des microbes. Ceci est particulièrement critique lorsque l'insert de pose et d'orientation est en matière plastique : les matières plastiques utilisables pour l'insert de pose et d'orientation, comme le polyéthylène par exemple, ont tendance à absorber les liquides. Et ceci est également particulièrement critique lorsque la cupule est en céramique, par exemple comme la cupule du document US 6,682,567 qui comporte des couches de céramique poreuse dont les pores peuvent absorber et retenir des liquides. Il y a donc un risque que les inserts de pose et d'orientation en matière plastique et/ou les cupules en céramique absorbent des liquides de condensats contaminés par des bactéries ou des microbes, ce qui entraîne alors une contamination du cotyle prothétique.

La structure de réception étant annulaire, elle permet l'orientation de la cupule selon n'importe quelle direction par l'application d'efforts radiaux sur l'impacteur selon toutes les directions possibles.

La structure de réception étant externe, la face de réception interne concave peut être impeccablement lisse pour un parfait glissement entre la cupule et la tête prothétique sphérique.

Avantageusement, le procédé peut en outre comporter une étape f) au cours de laquelle on stérilise l'ensemble ainsi formé et emballé dans une enveloppe de protection microbienne.

De préférence, on peut prévoir que l'étape f) de stérilisation est réalisée par bombardement de rayons gamma, de préférence selon une dose comprise entre environ 25 kGy et environ 40 kGy.

Afin de garantir une parfaite mobilité de la prothèse, il est important que la structure de réception ne limite pas le débattement du col prothétique.

De plus, le col prothétique portant la tête prothétique sphérique est généralement en une matière très résistante (telle que du métal par exemple) tandis que la céramique de la cupule est un matériau très dur mais fragile. Il en résulte que tout contact ou choc du col prothétique avec la céramique de la cupule endommagerait irrémédiablement celle-ci et doit donc être évité.

Pour ce faire, on peut avantageusement prévoir que :
- la face de réception interne concave est une portion de sphère comportant une face d'ouverture comprise dans un plan d'ouverture,
- la structure annulaire de réception est située en retrait du plan d'ouverture.

Du fait que la structure annulaire de réception pour la fixation de l'impacteur est située en retrait du plan d'ouverture, donc déportée en direction du sommet de la face extérieure d'ancrage convexe, cette structure annulaire de réception ne limite pas le débattement angulaire de la tige fémorale prothétique qui sera installée. Simultanément, on limite le risque de contact entre la tige fémorale prothétique et le bord de la cupule en céramique, ce qui réduit ainsi le risque de détérioration ou de bris de la cupule.

Avantageusement, on peut prévoir que :
- la structure annulaire externe de réception comprend un décrochement radial périphérique continu ou discontinu du bord annulaire, avec une face de réception orientée vers l'extérieur,
- l'insert de pose et d'orientation comporte une structure périphérique annulaire de fixation continue ou discontinue à face de liaison orientée vers l'intérieur, conformée pour coopérer avec la face de réception de la structure annulaire externe de réception.

Une telle structure annulaire externe de réception est relativement simple à fabriquer.

Selon une première variante, on peut prévoir que :
- la face de réception de la structure annulaire externe de réception comporte une gorge de verrouillage périphérique discontinue,
- la face de liaison de la structure périphérique annulaire de fixation de l'insert de pose et d'orientation comporte une pluralité de nervures de verrouillage réparties en périphérie et conformées pour s'engager dans la gorge de verrouillage périphérique discontinue.

Selon une seconde variante, on peut prévoir que :
- l'une de la face de réception de la structure annulaire externe de réception ou de la face de liaison de la structure périphérique annulaire de fixation comporte une gorge de verrouillage annulaire périphérique continue,
- l'autre de la face de liaison de la structure périphérique annulaire de fixation ou de la face de réception de la structure annulaire externe de réception comporte une nervure de verrouillage annulaire périphérique continue ou discontinue conformée pour s'engager dans la gorge de verrouillage annulaire périphérique continue.

La gorge de verrouillage et la ou les nervures de verrouillage participent à la retenue de l'insert de pose et d'orientation sur la cupule. On augmente également de façon importante la résistance de la liaison entre l'insert de pose et d'orientation et la cupule d'insertion pour endurer les couples d'orientation que le chirurgien transmet au moyen de l'impacteur.

Avantageusement, on peut prévoir que :
- le décrochement radial périphérique du bord annulaire a une épaisseur supérieure ou égale à environ 0,5 mm,
- ladite gorge de verrouillage périphérique discontinue ou ladite gorge de verrouillage annulaire périphérique continue de la structure annulaire externe de réception a une épaisseur radiale comprise entre environ 0,2 mm et environ 0,6 mm.

De telles dimensions sont compatibles avec une cupule en céramique de faible épaisseur au voisinage de son bord annulaire supérieur, par exemple d'environ 3 mm. En d'autres termes, ces dimensions sont telles qu'elles ne fragilisent pas ou peu la cupule en céramique au voisinage de son bord annulaire supérieur, tout en permettant une bonne tenue de l'insert de pose et d'orientation. L'utilisation d'une cupule de faible épaisseur au voisinage de son bord annulaire supérieur (par exemple une épaisseur voisine d'environ 3 mm) permet d'augmenter le diamètre de la tête sphérique de la partie mâle de l'articulation, et donc de limiter efficacement le risque de luxation.

De préférence, on peut prévoir que le décrochement radial périphérique du bord annulaire a une hauteur comprise entre environ 1 mm et environ 4 mm.

Une telle hauteur du décrochement radial périphérique procure une surface de contact suffisante entre l'insert de pose et d'orientation et la cupule en céramique pour le serrage radial, sans pour autant trop diminuer la surface de la face extérieure d'ancrage convexe destinée à venir en contact avec l'os. Ceci est d'autant plus important que la partie de face extérieure d'ancrage située sensiblement au voisinage du plan équatorial de celle-ci est une partie qui participe de façon importante à la retenue de la cupule dans la cavité cotyloïdienne du bassin.

Avantageusement, on peut prévoir que ladite gorge de verrouillage périphérique discontinue ou ladite gorge de verrouillage annulaire périphérique continue de la structure annulaire externe de réception a une hauteur comprise entre environ 0,4 mm et environ 3 mm.

De préférence, on peut prévoir que l'insert de pose et d'orientation et la structure annulaire externe de réception de la cupule sont conformés de telle sorte que, lorsque l'insert de pose et d'orientation est fixé à la structure annulaire externe de réception de la cupule, l'insert de pose et d'orientation ne dépasse pas à l'extérieur d'une surface sensiblement hémisphérique définie par la face extérieure d'ancrage convexe de la cupule. On évite ainsi tout risque de conflit de l'insert de pose et d'orientation avec la masse osseuse présente au voisinage de la cavité cotyloïdienne préparée du bassin du patient.

Avantageusement, la structure périphérique annulaire de fixation de l'insert de pose et d'orientation peut présenter une épaisseur radiale sensiblement égale ou inférieure à l'épaisseur du décrochement radial du bord annulaire.

De préférence, l'insert de pose et d'orientation peut être en polyéthylène. Le polyéthylène est d'usage courant dans le domaine médical, peu onéreux et facile à usiner. Le polyéthylène ne risque en outre pas d'endommager la cupule en céramique lorsque le chirurgien applique des chocs sur l'impacteur afin d'insérer la cupule dans la cavité cotyloïdienne du patient.

Avantageusement, l'insert de pose et d'orientation peut comporter une structure d'assemblage sur laquelle un impacteur peut être fixé de façon amovible.

De préférence, la structure d'assemblage peut comprendre un trou de fixation à taraudage interne pratiqué dans l'insert de pose et d'orientation, permettant le vissage d'une portion filetée correspondante de l'impacteur.

Avantageusement, le trou de fixation peut être un trou débouchant, apte à coopérer avec un outil de désolidarisation comportant une tige filetée apte à se visser dans le trou débouchant et qui a une extrémité distale conformée pour prendre appui contre la face de réception interne concave de la cupule lors du vissage de la tige filetée dans le trou débouchant.

De préférence, on peut prévoir que :
- l'insert de pose et d'orientation est conformé de façon à ce qu'il reste un espace libre entre l'insert de pose et d'orientation et le fond de la face de réception interne concave une fois l'insert de pose et d'orientation fixé à la structure annulaire externe de réception de la cupule,
- l'insert de pose et d'orientation est en contact de façon étanche selon sa face de liaison contre la face de réception de la structure annulaire externe de réception,
- le trou de fixation est un trou débouchant mettant en communication avec l'extérieur l'espace libre entre l'insert de pose et d'orientation et le fond de la face de réception interne concave et est dimensionné pour y engager l'extrémité d'une seringue de façon étanche.

### DESCRIPTION SOMMAIRE DES DESSINS

D'autres objets, caractéristiques et avantages de la présente invention ressortiront de la description suivante de variantes particulières, faite en relation avec les figures jointes, parmi lesquelles :
- la figure 4 est une vue en perspective d'une cupule en céramique d'un cotyle prothétique selon une première variante de l'invention ;
- la figure 5 est une vue de côté de la cupule en céramique de la figure 4 ;
- la figure 6 est une vue en perspective d'un insert de pose et d'orientation destiné à être associé avec la cupule en céramique des figures 4 et 5 ;
- la figure 7 est une vue en coupe de l'insert de pose et d'orientation de la figure 6 ;
- la figure 8 est une vue en perspective d'un insert de pose et d'orientation et d'une cupule en céramique d'un cotyle prothétique de hanche selon une deuxième variante de l'invention ;
- la figure 9 est une vue en coupe de l'insert de pose et d'orientation de la figure 8 ;
- la figure 10 est une vue partielle en coupe de la cupule en céramique de la figure 8;
- la figure 11 est une vue partielle en coupe de l'insert de pose et d'orientation de la figure 9 ;
- la figure 12 est une vue partielle en coupe de la cupule en céramique et de l'insert de pose et d'orientation des figures 10 et 11 ; et
- la figure 13 est une vue en coupe d'une cupule d'insertion et d'un insert de pose et d'orientation une fois assemblés.

Certaines figures jointes illustrent un objet qui ne fait pas partie de la présente invention mais qui est décrit ci-après et qui pourra faire l'objet d'une protection indépendante. Ces figures sont les suivantes :

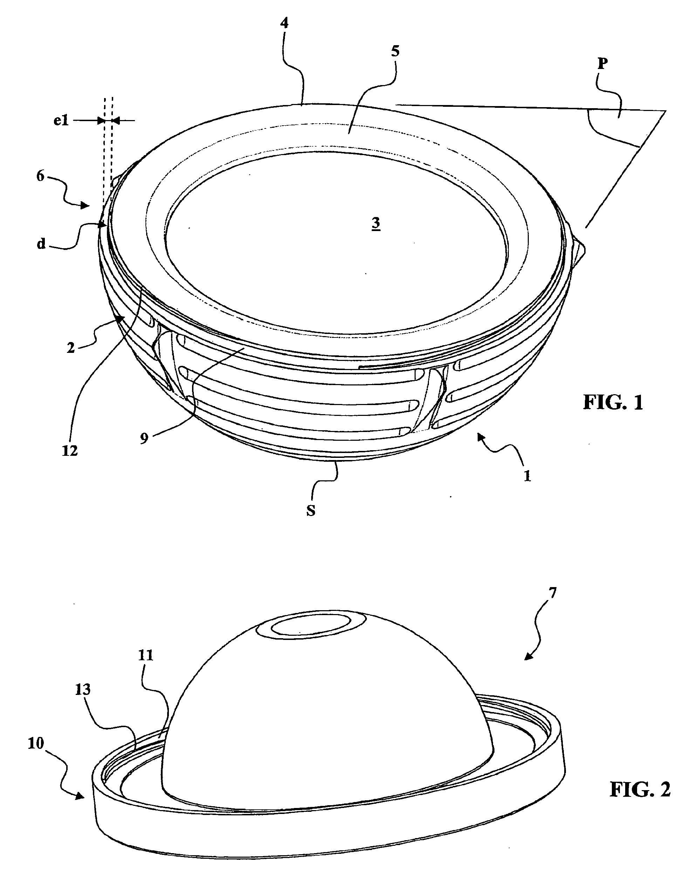

- la figure 1 qui est une vue en perspective d'une cupule en céramique d'un cotyle prothétique ;
- la figure 2 qui est une vue en perspective d'un insert de pose et d'orientation destiné à être associé à la cupule de la figure 1 ; et

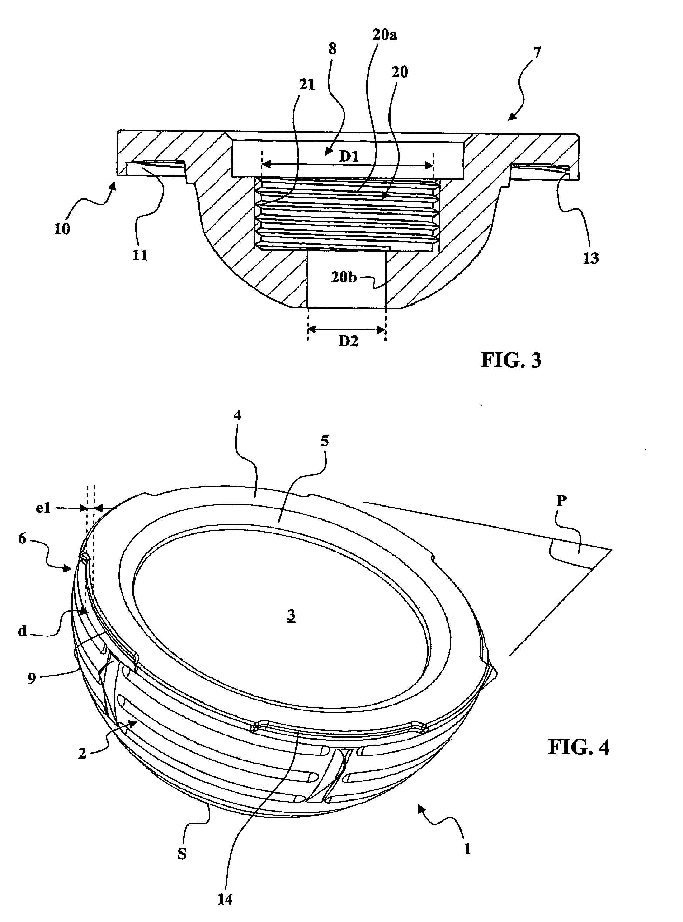

- la figure 3 qui est une vue en coupe de l'insert de pose et d'orientation de la figure 2.

### DESCRIPTION DES MODES DE REALISATION PREFERES

Sur chacune des figures 1, 4, 5 et 8 est représentée une cupule 1 en céramique de cotyle prothétique de hanche. Cette cupule 1 en céramique comprend :
- une face extérieure d'ancrage 2 convexe, conformée pour être ancrée dans une cavité cotyloïdienne du bassin d'un patient, et une face de réception interne 3 concave à bord annulaire 4,
- une structure annulaire de réception 6 est conformée de façon qu'un impacteur puisse être fixé à ladite structure annulaire de réception 6 pour l'impaction de la cupule 1 dans la cavité cotyloïdienne du bassin d'un patient.

La structure de réception 6 est externe, ce qui permet à la face de réception interne 3 concave d'être impeccablement lisse pour un parfait glissement entre la cupule et la tête prothétique sphérique.

On voit plus particulièrement que :
- la face de réception interne 3 concave est une portion de sphère comportant une face d'ouverture 5 comprise dans un plan d'ouverture P,
- la structure annulaire de réception 6 est située en retrait du plan d'ouverture P.

En d'autres termes, la structure annulaire de réception 6 est décalée par rapport au plan d'ouverture P en direction du sommet S de la face extérieure d'ancrage 2.

La structure annulaire de réception 6 ne se situe ainsi pas en prolongement de la face de réception interne 3 (qui est au plus hémisphérique) en prolongeant celle-ci à l'écart du sommet S. On évite ainsi de limiter le débattement angulaire du col prothétique, et on réduit le risque de contact entre la tige de col prothétique et le bord annulaire 4 de la cupule 1 en céramique.

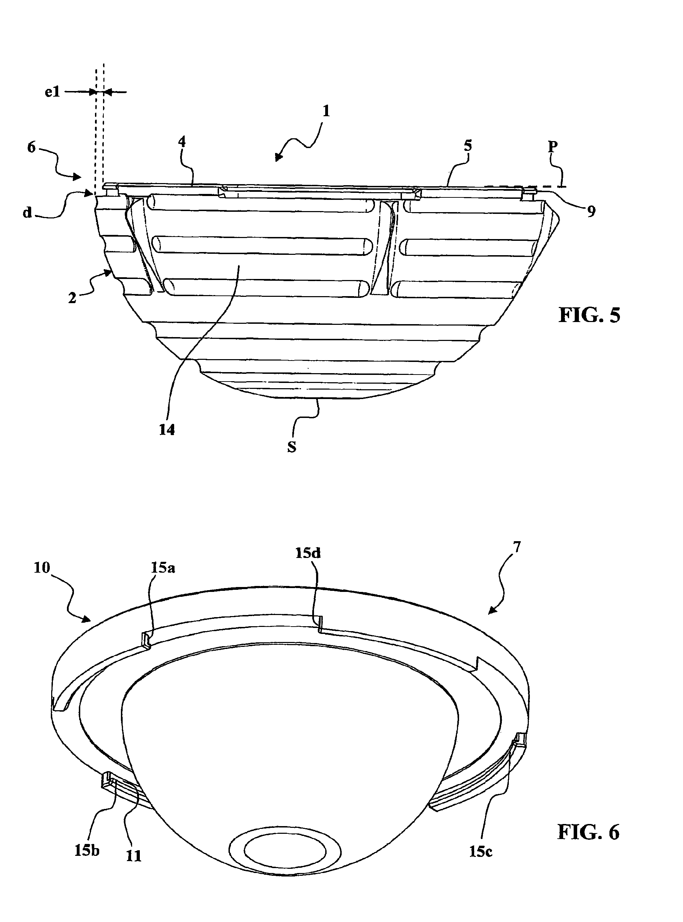

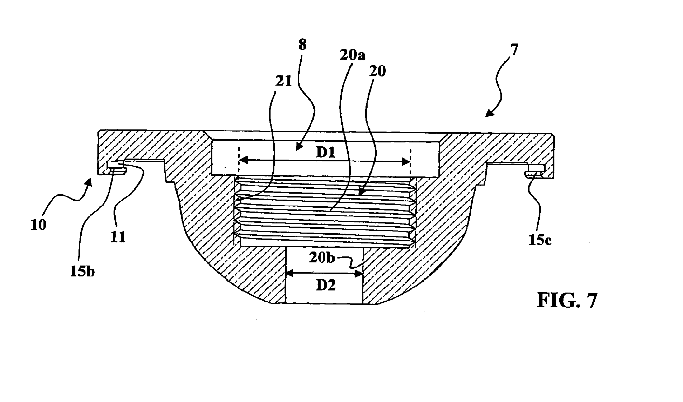

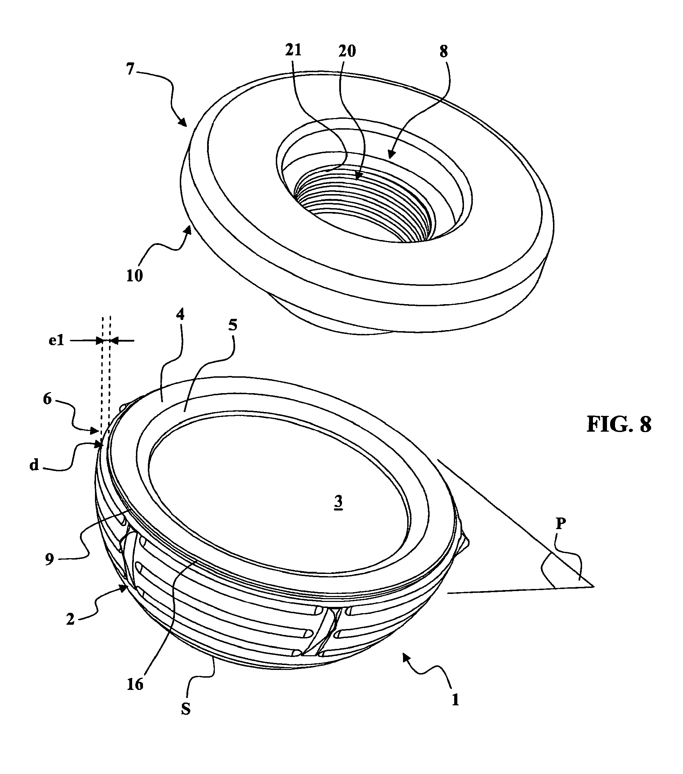

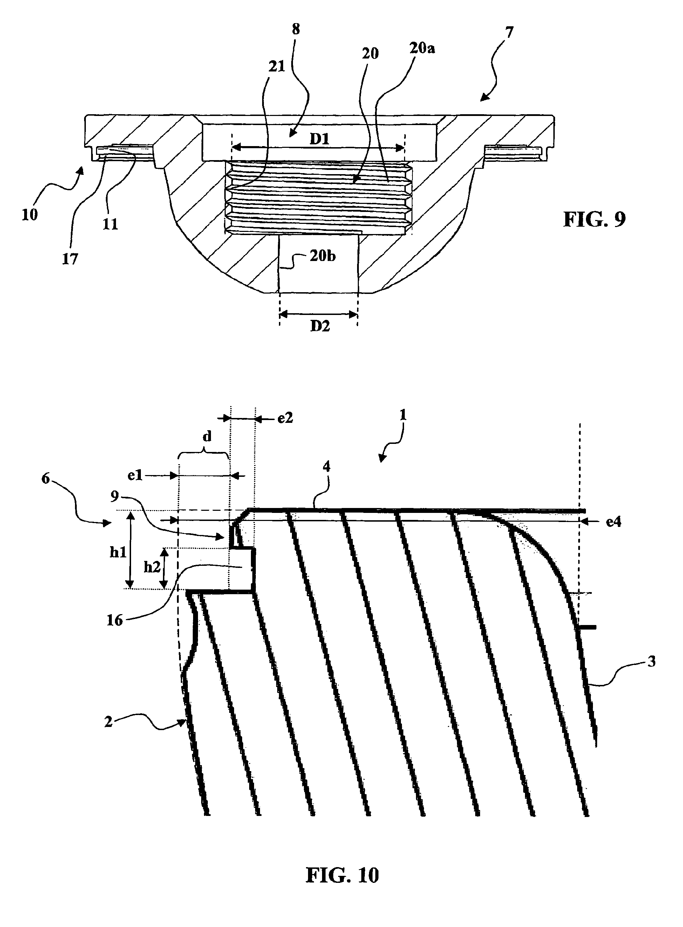

Sur les figures 2, 3, 6, 7 et 9 est représenté un insert de pose et d'orientation 7 destiné à être associé avec une cupule 1. L'insert de pose et d'orientation 7 des figures 2 et 3 est destiné à être associé à la cupule 1 de la figure 1. L'insert de pose et d'orientation 7 des figures 6 et 7 est destiné à être associé avec la cupule 1 des figures 4 et 5, ou 8. L'insert de pose et d'orientation 7 de la figure 9 est destiné à être associé avec la cupule 1 de la figure 8.

Chaque insert de pose et d'orientation 7 peut être fixé de façon amovible à la structure annulaire externe de réception 6 de la cupule 1 et comporte une structure d'assemblage 8 sur laquelle un impacteur peut être fixé de façon amovible.

Sur les figures 1, 5 et 8, on voit que la structure annulaire externe de réception 6 comprend un décrochement radial périphérique d continu ou discontinu du bord annulaire 4, avec une face de réception 9 orientée vers l'extérieur. On voit sur les figures 2, 3, 6, 7 et 9 que chaque insert de pose et d'orientation 7 comporte une structure périphérique annulaire de fixation 10 continue ou discontinue à face de liaison 11 orientée vers l'intérieur, conformée pour coopérer avec la face de réception 9 de la structure annulaire externe de réception 6.

Dans l'objet des figures 1 à 3, la face de réception 9 de la structure annulaire externe de réception 6 comporte un filetage externe 12 tandis que la face de liaison 11 de la structure périphérique annulaire de fixation 10 de l'insert de pose et d'orientation 7 comporte un filetage interne 13 conformé pour coopérer par vissage avec le filetage externe 12.

L'objet de la présente invention est illustré sur les figures 4 à 13. Sur ces figures, la structure annulaire externe de réception 6 et la structure périphérique annulaire de fixation 10 sont conformées de telle sorte que l'insert de pose et d'orientation 7 est engagé en force autour de la structure annulaire externe de réception 6 et y est retenu en force par un serrage radial de la structure périphérique annulaire de fixation 10 sur la structure annulaire externe de réception 6.

Du fait du caractère externe de la structure annulaire de réception 6, la surface de contact entre l'insert de pose et d'orientation 7 et la cupule 1 sur laquelle s'exerce le serrage est grande. Il en résulte une excellente fixation de l'insert de pose et d'orientation 7 sur la cupule 1.

Afin d'améliorer encore la tenue de l'insert de pose et d'orientation 7 sur la cupule 1, on peut prévoir une coopération supplémentaire entre l'insert de pose et d'orientation 7 et la cupule 1 au moyen d'une ou plusieurs nervures de verrouillage s'engageant dans une gorge de verrouillage.

Dans une première variante, illustrée sur les figures 4 à 7, on prévoit que :
- la face de réception 9 de la structure annulaire externe de réception 6 comporte une gorge de verrouillage 14 périphérique discontinue,
- la face de liaison 11 de la structure périphérique annulaire de fixation 10 de l'insert de pose et d'orientation 7 comporte une pluralité de nervures de verrouillage 15a à 15d réparties en périphérie et conformées pour s'engager dans la gorge de verrouillage 14 périphérique discontinue.

Dans une seconde variante, illustrée sur les figures 8 et 9, la face de réception 9 de la structure annulaire externe de réception 6 comporte une gorge de verrouillage 16 annulaire périphérique continue tandis que la face de liaison 11 de la structure périphérique annulaire de fixation 10 comporte une nervure de verrouillage 17 annulaire périphérique continue conformée pour s'engager dans la gorge de verrouillage 16 annulaire périphérique continue.

Dans cette deuxième variante, il est aussi possible que la face de liaison 11 de la structure périphérique annulaire de fixation 10 comporte une nervure de verrouillage annulaire périphérique discontinue. L'insert de pose et d'orientation 7 peut alors être semblable à celui de la première variante, illustrée sur les figures 6 et 7.

La figure 10 est une vue partielle en coupe du bord annulaire 4 de la cupule 1 de la figure 8. Sur cette figure 10, on voit plus particulièrement que la structure annulaire externe de réception 6 comprend un décrochement radial périphérique d avec une face de réception 9 orientée vers l'extérieur. Le décrochement radial périphérique d a une épaisseur e1 supérieure ou égale à environ 0,5 mm.

La face de réception 9 comporte la gorge de verrouillage 16 annulaire périphérique continue. La gorge de verrouillage 16 annulaire périphérique continue présente une épaisseur radiale e2 comprise entre environ 0,2 mm et environ 0,6 mm.

La cupule 1 en céramique comporte une épaisseur e4 au voisinage de son bord annulaire 4. L'épaisseur e4 peut être comprise entre 3 mm environ et 7 mm environ.

Le décrochement radial périphérique d du bord annulaire 4 a une hauteur h1 comprise entre environ 1 mm et environ 4 mm.

Enfin, la gorge de verrouillage 16 annulaire périphérique continue présente une hauteur h2 comprise entre environ 0,4 mm et 3 mm.

Toutes les dimensions énoncées ci-dessus en lien avec la deuxième variante sont valables pour la première variante ainsi que pour l'objet des figures 1 à 3.

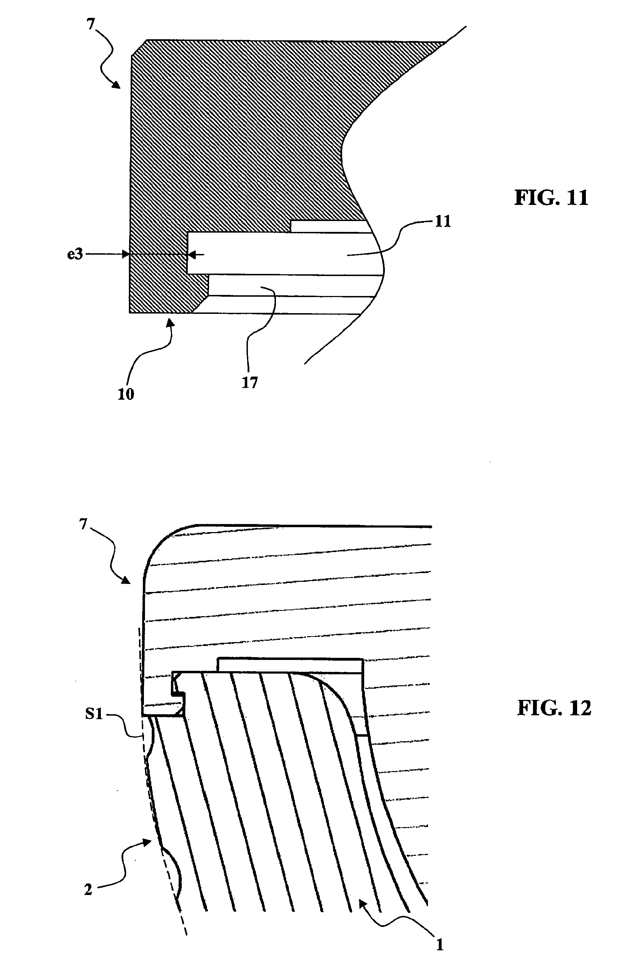

Comme on le voit sur la figure 11, la structure périphérique annulaire de fixation 10 de l'insert de pose et d'orientation 7 présente, avec sa face de liaison 11 et sa nervure de verrouillage 17 annulaire périphérique continue, une forme et des dimensions complémentaires de la face de réception 9 et de la gorge de verrouillage 16 annulaire périphérique continue. L'insert de pose et d'orientation 7 et la cupule 1 en céramique peuvent ainsi être associés de façon très intime, comme illustré sur la figure 12.

Afin d'éviter tout conflit de l'insert de pose et d'orientation 7 avec la masse osseuse autour de la cavité cotyloïdienne naturelle du bassin du patient, l'insert de pose et d'orientation 7 et la structure annulaire externe de réception 6 de la cupule 1 sont conformés de telle sorte que, lorsque l'insert de pose et d'orientation 7 est fixé à la structure annulaire externe de réception 6 de la cupule 1, l'insert de pose et d'orientation 7 ne dépasse pas à l'extérieur d'une surface sensiblement hémisphérique S1 (illustrée en pointillés sur la figure 12) définie par la face extérieure d'ancrage 2 convexe de la cupule 1. La face extérieure d'ancrage 2 est conformée de façon sensiblement hémisphérique afin de s'adapter et se fixer de façon fiable dans la cavité cotyloïdienne du patient.

Pour ce faire, la structure périphérique annulaire de fixation 10 de l'insert de pose et d'orientation 7 présente une épaisseur radiale e3 (figure 11) sensiblement égale ou inférieure à l'épaisseur e1 du décrochement radial d du bord annulaire 4 (figure 10).

Afin de ne pas risquer d'endommager la cupule 1 en céramique, l'insert de pose et d'orientation 7 est avantageusement fabriqué en matière plastique, de préférence en polyéthylène qui est une matière peu onéreuse, facile à usiner et qui évite d'endommager la céramique de la cupule 1 malgré l'application de chocs sur l'impacteur lors de l'insertion de la cupule 1 dans la cavité cotyloïdienne du patient.

Dans le cadre de l'invention, la face de réception interne 3 concave peut être une face de glissement 3a pour la réception de la tête fémorale prothétique. La face de réception interne 3 concave peut également être une face de glissement 3a pour la réception d'un insert articulaire mobile, l'insert articulaire mobile recevant à pivotement la tête fémorale prothétique, pour un cotyle à double mobilité par exemple.

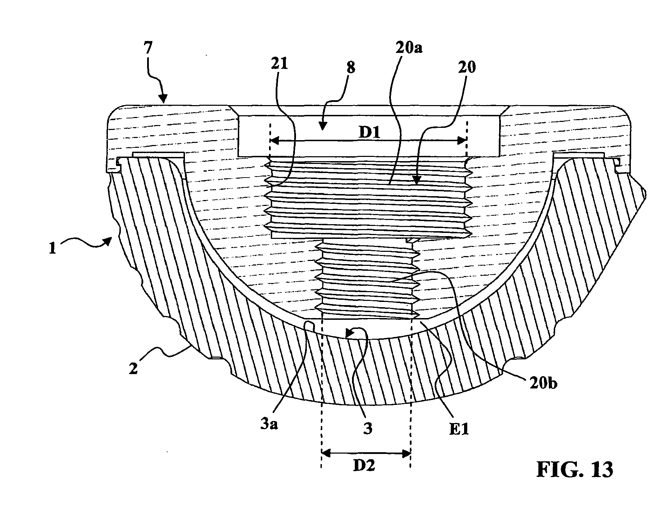

On voit plus particulièrement sur la figure 13 que la structure d'assemblage 8 de l'insert de pose et d'orientation 7 comprend un trou de fixation 20 à taraudage interne 21. Le taraudage interne 21 permet le vissage d'une portion filetée correspondante de l'impacteur (non représenté).

Après l'impaction de la cupule 1 en céramique dans la cavité cotyloïdienne du bassin du patient, il est nécessaire de procéder au retrait de l'insert de pose et d'orientation 7. Il est nécessaire que ce retrait s'effectue sans induire d'effort ou de contrainte sensible entre la face extérieure d'ancrage 2 et la cavité cotyloïdienne du bassin du patient pour ne pas détruire ou dégrader la liaison réalisée entre celles-ci lors de l'impaction.

Une première solution de séparation consiste alors à prévoir que le trou de fixation 20 est un trou débouchant, apte à coopérer avec un outil de désolidarisation comportant une tige filetée à extrémité distale conformée pour prendre appui contre la face de réception interne 3 concave de la cupule 1 lors du vissage de la tige filetée dans le trou débouchant. Sur la figure 13, le trou de fixation 20 comporte un premier tronçon 20a de diamètre D1 et un second tronçon 20b de diamètre D2 plus petit. La tige filetée de l'outil de désolidarisation présente un diamètre extérieur adapté pour que celle-ci vienne se visser dans le tronçon 20b de diamètre D2 du trou de fixation 20 débouchant.

En alternative, la tige filetée de l'outil de désolidarisation peut être logée à coulissement à l'intérieur de l'impacteur (qui vient se visser dans le tronçon 20a) et présente alors un diamètre inférieur au diamètre D2 du tronçon 20b afin de venir prendre appui contre la face de réception interne 3 en passant à travers le tronçon 20b.

Une seconde solution de séparation consiste à prévoir que :
- l'insert de pose et d'orientation 7 est conformé de façon à ce qu'il reste un espace libre E1 entre l'insert de pose et d'orientation 7 et le fond de la face de réception interne 3 concave (figure 13) une fois l'insert de pose et d'orientation 7 fixé à la structure annulaire externe de réception 6 de la cupule 1,
- l'insert de pose et d'orientation 7 est en contact de façon étanche selon sa face de liaison 11 contre la face de réception 9 de la structure annulaire externe de réception 6,
- le trou de fixation 20 est un trou débouchant mettant en communication avec l'extérieur l'espace libre E1 (prévu entre l'insert de pose et d'orientation 7 et le fond de la face de réception interne 3 concave) et est dimensionné pour y engager l'extrémité d'une seringue de façon étanche.

Dans le cas de la figure 13, l'extrémité de seringue présente un diamètre extérieur adapté pour venir s'engager de façon étanche dans le tronçon de diamètre D2 du trou de fixation 20.

Par actionnement de la seringue, on injecte ainsi sous pression un liquide tel que de l'eau ou un sérum physiologique dans l'espace libre E1 pour induire une force de séparation entre la cupule 1 et l'insert de pose et d'orientation 7.

Afin d'obtenir un contact étanche entre l'insert de pose et d'orientation 7 et la cupule 1, on utilisera de préférence la cupule d'insertion 1 et l'insert de pose et d'orientation 7 illustrés sur les figures 8 et 9.

Dans le cadre de la présente invention, lors de l'assemblage de l'ensemble comprenant la cupule 1 et un insert de pose et d'orientation 7, il est important de ne pas dégrader la structure annulaire de réception 6 et la structure périphérique annulaire de fixation 10. Or, afin de réaliser un serrage radial de la structure périphérique annulaire de fixation 10 sur la structure annulaire externe de réception 6, il est nécessaire que la structure périphérique annulaire de fixation 10 présente des dimensions diamétrales égales ou inférieures à celles de la structure annulaire externe de réception 6.

On a alors recours à un procédé d'assemblage par dilatation puis retrait au cours duquel on chauffe l'insert de pose et d'orientation 7 afin d'en augmenter les dimensions, puis on engage la structure périphérique annulaire de fixation 10 autour de la face de réception 9 de la structure annulaire externe de réception 6, et on ramène enfin à température ambiante l'insert de pose et d'orientation 7 pour en diminuer les dimensions.

Un tel procédé permet un assemblage sans émousser ni endommager la ou les nervures de verrouillage 15a-15d ou 17, qui conservent ainsi des arêtes vives pour une liaison plus forte entre l'insert de pose et d'orientation 7 et la cupule 1 en céramique.

Ce procédé de fabrication par chauffage n'induit aucun risque de contamination de l'insert de pose et d'orientation 7, notamment lorsque celui-ci est en polyéthylène.

Lors de son retour à température ambiante, il se produit un serrage radial de la structure périphérique annulaire de fixation 10 sur la structure annulaire externe de réception 6.

On peut ensuite procéder à une étape de stérilisation de l'ensemble ainsi formé et emballé dans une enveloppe de protection microbienne. Une stérilisation satisfaisante sera obtenue par bombardement de rayons gamma, de préférence selon une dose comprise entre environ 25 kGy et environ 40 kGy.

Il est expressément souligné que le procédé de fabrication et d'assemblage de l'ensemble de la figure 13 par chauffage de l'insert de pose et d'orientation 7 puis retour à température ambiante peut être utilisé avec une cupule 1 en céramique dont la structure annulaire externe de réception 6 n'est pas obligatoirement située en retrait du plan d'ouverture P. Le procédé de fabrication pourra ainsi faire l'objet d'une mise en oeuvre quelle que soit la position de la structure annulaire externe de réception 6 par rapport au plan d'ouverture P de la cupule 1.

La présente invention n'est pas limitée aux modes de réalisation qui ont été explicitement décrits, mais elle en inclut les diverses variantes et généralisations contenues dans le domaine des revendications ci-après.

## Revendications

1. Procédé de fabrication d'un cotyle prothétique de hanche, comprenant les étapes de :
a) prévoir une cupule (1) en céramique ayant une face de réception interne (3) concave et ayant une structure annulaire de réception externe (6),
b) prévoir un insert de pose et d'orientation (7) à structure périphérique annulaire de fixation (10) conformée pour coopérer avec la face de réception (9) de la structure annulaire externe de réception (6) en s'engageant autour de la structure annulaire externe de réception (6),
c) chauffer l'insert de pose et d'orientation (7) afin d'en augmenter les dimensions,
d) engager la structure périphérique annulaire de fixation (10) autour de la face de réception (9) de la structure annulaire externe de réception (6),
e) ramener à température ambiante l'insert de pose et d'orientation (7) pour en diminuer les dimensions de façon à obtenir un serrage radial de la structure périphérique annulaire de fixation (10) sur la structure annulaire externe de réception (6).

2. Procédé selon la revendication 1, **caractérisé en ce que** :
- la face de réception interne (3) concave est une portion de sphère comportant une face d'ouverture (5) comprise dans un plan d'ouverture (P),
- la structure annulaire de réception (6) est située en retrait du plan d'ouverture (P).

3. Procédé selon l'une des revendications 1 ou 2, **caractérisé en ce qu'**il comporte en outre une étape f) au cours de laquelle on stérilise l'ensemble ainsi formé et emballé dans une enveloppe de protection microbienne.

4. Procédé selon la revendication 3, **caractérisé en ce que** l'étape f) de stérilisation est réalisée par bombardement de rayons gamma, de préférence selon une dose comprise entre environ 25 kGy et environ 40 kGy.

5. Procédé selon l'une quelconque des revendications 1 à 4, **caractérisé en ce que** l'insert de pose et d'orientation (7) est en polyéthylène.

6. Procédé selon l'une quelconque des revendications 1 à 5, **caractérisé en ce que** :
- la structure annulaire externe de réception (6) comprend un décrochement radial périphérique (d) continu ou discontinu du bord annulaire (4), avec une face de réception (9) orientée vers l'extérieur,
- l'insert de pose et d'orientation (7) comporte une structure périphérique annulaire de fixation (10) continue ou discontinue à face de liaison (11) orientée vers l'intérieur, conformée pour coopérer avec la face de réception (9) de la structure annulaire externe de réception (6).

7. Procédé selon la revendication 6, **caractérisé en ce que** :
- la face de réception (9) de la structure annulaire externe de réception (6) comporte une gorge de verrouillage (14) périphérique discontinue,
- la face de liaison (11) de la structure périphérique annulaire de fixation (10) de l'insert de pose et d'orientation (7) comporte une pluralité de nervures de verrouillage (15a-15d) réparties en périphérie et conformées pour s'engager dans la gorge de verrouillage (14) périphérique discontinue.

8. Procédé selon la revendication 6, **caractérisé en ce que** :
- l'une de la face de réception (9) de la structure annulaire externe de réception (6) ou de la face de liaison (11) de la structure périphérique annulaire de fixation (10) comporte une gorge de verrouillage (16) annulaire périphérique continue,
- l'autre de la face de liaison (11) de la structure périphérique annulaire de fixation (10) ou de la face de réception (9) de la structure annulaire externe de réception (6) comporte une nervure de verrouillage (17) annulaire périphérique continue ou discontinue conformée pour s'engager dans la gorge de verrouillage (16) annulaire périphérique continue.

9. Procédé selon l'une quelconque des revendications 7 ou 8, **caractérisé en ce que** :
- le décrochement radial périphérique (d) du bord annulaire (4) a une épaisseur (e1) supérieure ou égale à environ 0,5 mm,
- ladite gorge de verrouillage (14) périphérique discontinue ou ladite gorge de verrouillage (16) annulaire périphérique continue de la structure annulaire externe de réception (6) a une épaisseur radiale (e2) comprise entre environ 0,2 mm et environ 0,6 mm.

10. Procédé selon l'une quelconque des revendications 7 à 9, **caractérisé en ce que** ladite gorge de verrouillage (14) périphérique discontinue ou ladite gorge de verrouillage (16) annulaire périphérique continue de la structure annulaire externe de réception (6) a une hauteur (h2) comprise entre environ 0,4 mm et environ 3 mm.

11. Procédé selon l'une quelconque des revendications 6 à 10, **caractérisé en ce que** le décrochement radial périphérique (d) du bord annulaire (4) a une hauteur (h1) comprise entre environ 1 mm et environ 4 mm.

12. Procédé selon l'une quelconque des revendications 6 à 11, **caractérisé en ce que** l'insert de pose et d'orientation (7) et la structure annulaire externe de réception (6) de la cupule (1) sont conformés de telle sorte que, lorsque l'insert de pose et d'orientation (7) est fixé à la structure annulaire externe de réception (6) de la cupule (1), l'insert de pose et d'orientation (7) ne dépasse pas à l'extérieur d'une surface sensiblement hémisphérique (S1) définie par la face extérieure d'ancrage (2) convexe de la cupule (1).

13. Procédé selon la revendication 12, **caractérisé en ce que** la structure périphérique annulaire de fixation (10) de l'insert de pose et d'orientation (7) présente une épaisseur (e3) radiale sensiblement égale ou inférieure à l'épaisseur (e1) du décrochement radial (d) du bord annulaire (4).

14. Procédé selon l'une quelconque des revendications 1 à 13, **caractérisé en ce que** l'insert de pose et d'orientation (7), destiné à être fixé de façon amovible à la structure annulaire externe de réception (6) de la cupule (1), comporte une structure d'assemblage (8) sur laquelle un impacteur peut être fixé de façon amovible.

15. Procédé selon la revendication 14, **caractérisé en ce que** la structure d'assemblage (8) comprend un trou de fixation (20) à taraudage interne (21) pratiqué dans l'insert de pose et d'orientation (7), permettant le vissage d'une portion filetée correspondante de l'impacteur.

16. Procédé selon la revendication 15, **caractérisé en ce que** le trou de fixation (20) est un trou débouchant, apte à coopérer avec un outil de désolidarisation comportant une tige filetée apte à se visser dans le trou débouchant et qui a une extrémité distale conformée pour prendre appui contre la face de réception interne (3) concave de la cupule (1) lors du vissage de la tige filetée dans le trou débouchant.

17. Procédé selon la revendication 15, **caractérisé en ce que** :
- l'insert de pose et d'orientation (7) est conformé de façon à ce qu'il reste un espace libre (E1) entre l'insert de pose et d'orientation (7) et le fond de la face de réception interne (3) concave une fois l'insert de pose et d'orientation (7) fixé à la structure annulaire externe de réception (6) de la cupule (1),
- l'insert de pose et d'orientation (7) est en contact de façon étanche selon sa face de liaison (11) contre la face de réception (9) de la structure annulaire externe de réception (6),
- le trou de fixation (20) est un trou débouchant mettant en communication avec l'extérieur l'espace libre (E1) entre l'insert de pose et d'orientation (7) et le fond de la face de réception interne (3) concave et est dimensionné pour y engager l'extrémité d'une seringue de façon étanche.

18. Procédé selon l'une quelconque des revendications 1 à 17, **caractérisé en ce que** la cupule (1) comporte une épaisseur (e4) au voisinage de son bord annulaire (4) comprise entre 3 mm environ et 7 mm environ.

## Patentansprüche

1. Verfahren zum Herstellen einer Hüftgelenkpfannenprothese, umfassend die Schritte:
a) Bereitstellen einer Schale (1) aus Keramik, die eine konkave innere Aufnahmefläche (3) aufweist, und eine äußere ringförmige Aufnahmestruktur (6) aufweist,
b) Bereitstellen eines Installations- und Ausrichtungseinsatzes (7), der eine ringförmige Befestigungsstruktur (10) aufweist, die dazu vorgesehen ist, mit der Aufnahmefläche (9) der äußeren ringförmigen Aufnahmestruktur (6) durch Verkuppeln um die äußere ringförmige Aufnahmestruktur (6) zusammenzuwirken,
c) Erhitzen des Installations- und Ausrichtungseinsatzes (7), um seine geometrische Ausdehnung zu erhöhen,
d) Verkuppeln der ringförmigen Befestigungsstruktur (10) um die Aufnahmefläche (9) der äußeren ringförmigen Aufnahmestruktur (6),
e) Rückführen des Installations- und Ausrichtungseinsatzes (7) auf Umgebungstemperatur um seine geometrische Ausdehnung zu reduzieren, so dass ein radiales Verspannen der ringförmigen Befestigungsstruktur (10) auf der äußeren ringförmigen Aufnahmestruktur (6) erreicht wird.

2. Verfahren nach Anspruch 1, **dadurch gekennzeichnet, dass**:
- die konkave innere Aufnahmefläche (3) ein Teil einer Kugelfläche ist, die eine Öffnungsseite (5) aufweist, die in einer Öffnungsebene (P) enthalten ist,
- die ringförmige Aufnahmestruktur (6) gegenüber der Öffnungsebene (P) zurückgesetzt gelegen ist.

3. Verfahren nach einem der Ansprüche 1 oder 2, **dadurch gekennzeichnet, dass** es zusätzlich einen Schritt f) enthält, während dessen Ausführung man die so gebildete und in einer mikrobiologischen Schutzhülle gepackte Einheit sterilisiert.

4. Verfahren nach Anspruch 3, **dadurch gekennzeichnet, dass** der Schritt f) der Sterilisation durch Bestrahlen mit Gammastrahlen vorzugsweise bei einer Dosis zwischen ungefähr 25 kGy und ungefähr 40 kGy durchgeführt wird.

5. Verfahren nach irgendeinem der Ansprüche 1-4, **dadurch gekennzeichnet, dass** der Installations- und Ausrichtungseinsatz (7) aus Polyethylen gefertigt ist.

6. Verfahren nach irgendeinem der Ansprüche 1-5, **dadurch gekennzeichnet, dass**:
- die äußere ringförmige Aufnahmestruktur (6) eine durchgehende oder unterbrochene, umlaufende radiale Schulter (d) eines ringförmigen Randes (4) aufweist mit einer Aufnahmefläche (9), die zur Außenseite gerichtet ist,
- der Installations- und Ausrichtungseinsatz (7) eine durchgehende oder unterbrochene ringförmige Befestigungsstruktur (10) mit einer Verbindungsfläche (11) aufweist, die zur Innenseite gerichtet und vorgesehen ist, mit der Aufnahme-fläche (9) der äußeren ringförmigen Aufnahmestruktur (6) zusammenzuwirken.

7. Verfahren nach Anspruch 6, **dadurch gekennzeichnet, dass**:
- die Aufnahmefläche (9) der äußeren ringförmigen Aufnahmestruktur (6) eine unterbrochene umfänglich verlaufende Verriegelungskehle (14) aufweist,
- die Verbindungsfläche (11) der umfänglich verlaufenden ringförmigen Befestigungsstruktur (10) des Installations- und Ausrichtungseinsatzes (7) eine Vielzahl von Verriegelungsrippen (15a-15d) aufweist, die umfänglich verteilt und vorgesehen sind, in die umfänglich verlaufende Verriegelungskehle (14) einzugreifen.

8. Verfahren nach Anspruch 6, **dadurch gekennzeichnet, dass**:
- eine der Aufnahmeflächen (9) der äußeren ringförmigen Aufnahmestruktur (6) oder der Verbindungsfläche (11) der umfänglich verlaufenden ringförmigen Befestigungsstruktur (10) eine durchgehende umfänglich verlaufende Verriegelungskehle (16) aufweist,
- die andere der Verbindungsfläche (11) der umfänglich verlaufenden ringförmigen Befestigungsstruktur (10) oder der Aufnahmefläche (9) der äußeren ringförmigen Aufnahmestruktur (6) eine durchgehende oder unterbrochene umfänglich verlaufende ringförmige Verriegelungsrippe (17) aufweist, die vorgesehen ist, in die durchgehende umfänglich verlaufende Verriegelungskehle (16) einzugreifen.

9. Verfahren nach einem der Ansprüche 7 oder 8, **dadurch gekennzeichnet, dass**:
- die umfänglich verlaufende radiale Schulter (d) der ringförmigen Kante (4) eine Dicke (e1) von größer oder gleich ungefähr 0,5 mm aufweist,
- die unterbrochene umfänglich verlaufende Verriegelungskehle (14) oder die durchgehende umfänglich verlaufende ringförmige Verriegelungskehle (16) der äußeren ringförmigen Aufnahmestruktur (6) eine radiale Dicke (e2) zwischen ungefähr 0,2 mm und ungefähr 0,6 mm aufweist.

10. Verfahren nach einem der Ansprüche 7 bis 9, **dadurch gekennzeichnet, dass** die unterbrochene umfänglich verlaufende Verriegelungskehle (14) oder die durchgehende umfänglich verlaufende ringförmige Verriegelungskehle (16) der äußeren ringförmigen Aufnahmestruktur (6) eine Höhe (h2) zwischen ungefähr 0,4 mm und ungefähr 3 mm aufweisen.

11. Verfahren nach einem der Ansprüche 6 bis 10, **dadurch gekennzeichnet, dass** die umfänglich verlaufende radiale Schulter (d) der ringförmigen Kante (4) eine Höhe (h1) zwischen ungefähr 1 mm und ungefähr 4 mm aufweist.

12. Verfahren nach einem der Ansprüche 6 bis 11, **dadurch gekennzeichnet, dass** der Installations-und Ausrichtungseinsatz (7) und die ringförmige äußere Aufnahmestruktur (6) der Schale (1) derart ausgelegt sind, dass wenn der Installations- und Ausrichtungseinsatz (7) an der äußeren ringförmigen Aufnahmestruktur (6) der Schale (1) befestigt ist, der Installations- und Ausrichtungseinsatz (7) nicht vor eine im wesentlichen halbkugelförmige Fläche (S1) ragt, die durch die äußere konvexe Verankerungsfläche (2) der Schale (1) vorgegeben ist.

13. Verfahren nach Anspruch 12, **dadurch gekennzeichnet, dass** die umfänglich verlaufende ringförmige Befestigungsstruktur (10) des Installations- und Ausrichtungseinsatzes (7) eine radiale Dicke (e3) aufweist, die im wesentlichen gleich oder kleiner als die Dicke (e1) der radialen Schulter (d) des ringförmigen Randes (4) ist.

14. Verfahren nach einem der Ansprüche 1 bis 13, **dadurch gekennzeichnet, dass** der Installations-und Ausrichtungseinsatz (7), der dazu bestimmt ist, in unbeweglicher Form an der ringförmigen externen Aufnahmestruktur (6) der Schale (1) befestigt zu werden, eine Montagestruktur (8) aufweist, an der ein Impaktor lösbar fixiert werden kann.

15. Verfahren nach Anspruch 14, **dadurch gekennzeichnet, dass** die Montagestruktur (8) eine im Installations- und Ausrichtungseinsatz (7) ausgebildete Befestigungsbohrung (20) mit einem Innengewinde (21) umfasst, die ein Verschrauben eines entsprechenden Gewindebereiches des Impaktors erlaubt.

16. Verfahren nach Anspruch 15, **dadurch gekennzeichnet, dass** die Befestigungsbohrung (20) eine Durchgangsbohrung ist, die mit einem Lösewerkzeug zusammenwirken kann, das einen Gewindestab umfasst, der in die Durchgangsbohrung eingeschraubt werden kann und der ein äußeres Ende aufweist, das eingerichtet ist, gegen die konkave innere Aufnahmefläche (3) der Schale (1) in Anschlag zu kommen, wenn der Gewindestab in die Durchgangsbohrung eingeschraubt wird.

17. Verfahren nach Anspruch 15, **dadurch gekennzeichnet, dass**:
- der Installations- und Ausrichtungseinsatz (7) derart ausgebildet ist, dass ein freier Raum (E1) zwischen dem Installations- und Ausrichtungseinsatz (7) und dem Boden der konkaven inneren Aufnahmefläche (3) verbleibt, sobald der Installations- und Ausrichtungseinsatz (7) an der äußeren ringförmigen Aufnahmestruktur (6) der Schale (1) fixiert ist,
- der Installations- und Ausrichtungseinsatz (7) in dichter Weise mit seiner Verbindungsfläche (11) gegen die Aufnahmefläche (9) der ringförmigen externen Aufnahmestruktur (6) in Kontakt ist,
- die Befestigungsbohrung (20) eine Durchgangsbohrung ist, die mit dem äußeren freien Raum (E1) zwischen dem Installations- und Ausrichtungseinsatz (7) und dem Boden der konkaven inneren Aufnahmefläche (3) eine Verbindung herstellt und dimensioniert ist, um dadurch in dichter Weise das Ende einer Spritze zu halten.

18. Verfahren nach einem der Ansprüche 1 bis 17, **dadurch gekennzeichnet, dass** die Schale (1) nahe ihrem ringförmigen Rand (4) eine Dicke (e4) zwischen ungefähr 3 mm und ungefähr 7 mm aufweist.

## Claims

1. Method for producing a prosthetic hip acetabulum, comprising the steps of:
a) providing a ceramic cup (1) having a concave inner receiving face (3) and having an outer annular receiving structure (6),
b) providing an installation and orientation insert (7) having a peripheral annular fixing structure (10) designed to cooperate with the receiving face (9) of the outer annular receiving structure (6) by engaging around the outer annular receiving structure (6),
c) heating the installation and orientation insert (7) in order to increase the dimensions thereof,
d) engaging the peripheral annular fixing structure (10) around the receiving face (9) of the outer annular receiving structure (6),
e) bringing the installation and orientation insert (7) back to room temperature in order to reduce the dimensions thereof, so as to achieve a radial clamping of the peripheral annular fixing structure (10) on the outer annular receiving structure (6).

2. Method as claimed in claim 1, **characterized in that**:
- the concave inner receiving face (3) is a sphere portion having an opening face (5) contained in an opening plane (P),
- the annular receiving structure (6) is set back from the opening plane (P).

3. Method as claimed in either of claims 1 and 2, **characterized in that** it additionally has a step f) during which the unit thus formed and packed in a microbial protection envelope is sterilized.

4. Method as claimed in claim 3, **characterized in that** step f) of sterilization is carried out by bombardment with gamma rays, preferably at a dose of between approximately 25 kGy and approximately 40 kGy.

5. Method as claimed in any of claims 1 through 4, **characterized in that** the installation and orientation insert (7) is made of polyethylene.

6. Method as claimed in any of claims 1 through 5, **characterized in that**:
- the outer annular receiving structure (6) comprises a continuous or interrupted peripheral radial shoulder (d) of the annular edge (4), with a receiving face (9) directed toward the outside,
- the installation and orientation insert (7) has a continuous or interrupted peripheral annular fixing structure (10) with a connecting face (11) directed toward the inside and designed to cooperate with the receiving face (9) of the outer annular receiving structure (6).

7. Method as claimed in claim 6, **characterized in that**:
- the receiving face (9) of the outer annular receiving structure (6) has an interrupted peripheral locking groove (14),
- the connecting face (11) of the peripheral annular fixing structure (10) of the installation and orientation insert (7) has a plurality of locking ribs (15a-15d) which are distributed peripherally and are designed so as to engage in the interrupted peripheral locking groove (14).

8. Method as claimed in claim 6, **characterized in that**:
- either the receiving face (9) of the outer annular receiving structure (6) or the connecting face (11) of the peripheral annular fixing structure (10) has a continuous peripheral annular locking groove (16),
- the other of the connecting face (11) of the peripheral annular fixing structure (10) or the receiving face (9) of the outer annular receiving structure (6) has a continuous or interrupted peripheral annular locking rib (17) designed so as to engage in the continuous peripheral annular locking groove (16).

9. Method as claimed in either of claims 7 and 8, **characterized in that**:
- the peripheral radial shoulder (d) of the annular edge (4) has a thickness (e1) of greater than or equal to approximately 0.5 mm,
- said interrupted peripheral locking groove (14) or said continuous peripheral annular locking groove (16) of the outer annular receiving structure (6) has a radial thickness (e2) of between approximately 0.2 mm and approximately 0.6 mm.

10. Method as claimed in any of claims 7 through 9, **characterized in that** said interrupted peripheral locking groove (14) or said continuous peripheral annular locking groove (16) of the outer annular receiving structure (6) has a height (h2) of between approximately 0.4 mm and approximately 3 mm.

11. Method as claimed in any of claims 6 through 10, **characterized in that** the peripheral radial shoulder (d) of the annular edge (4) has a height (h1) of between approximately 1 mm and approximately 4 mm.

12. Method as claimed in any of claims 6 through 11, **characterized in that** the installation and orientation insert (7) and the outer annular receiving structure (6) of the insertion cup (1) are designed in such a way that, when the installation and orientation insert (7) is fixed to the outer annular receiving structure (6) of the insertion cup (1), the installation and orientation insert (7) does not protrude outside a substantially hemispherical surface (S1) defined by the convex outer anchoring face (2) of the cup (1).

13. Method as claimed in claim 12, **characterized in that** the peripheral annular fixing structure (10) of the installation and orientation insert (7) has a radial thickness (e3) substantially equal to or less than the thickness (e1) of the radial shoulder (d) of the annular edge (4).

14. Method as claimed in any of claims 1 through 13, **characterized in that** the installation and orientation insert (7), intended to be fixed removably to the outer annular receiving structure (6) of the cup (1), has an assembly structure (8) on which an impactor can be fixed removably.

15. Method as claimed in claim 14, **characterized in that** the assembly structure (8) comprises a fixation hole (20) with internal thread (21) made in the installation and orientation insert (7), permitting the screwing of a corresponding threaded portion of the impactor.

16. Method as claimed in claim 15, **characterized in that** the fixation hole (20) is a through-hole able to cooperate with a disconnecting tool having a threaded rod which is able to be screwed into the through-hole and which has a distal end designed to bear against the concave inner receiving face (3) of the cup (1) when the threaded rod is screwed into the through-hole.

17. Method as claimed in claim 15, **characterized in that**:
- the installation and orientation insert (7) is designed in such a way that a free space (E1) remains between the installation and orientation insert (7) and the bottom of the concave inner receiving face (3) once the installation and orientation insert (7) has been fixed to the outer annular receiving structure (6) of the insertion cup (1),
- the installation and orientation insert (7) bears in a sealed manner across its connecting face (11) against the receiving face (9) of the outer annular receiving structure (6),
- the fixation hole (20) is a through-hole via which the free space (E1) between the installation and orientation insert (7) and the bottom of the concave inner receiving face (3) is brought into communication with the outside and which is dimensioned for leaktight engagement of the end of a syringe.

18. Method as claimed in any of claims 1 through 17, **characterized in that** the cup (1) has a thickness (e4) near its annular edge (4) comprised between approximately 3 mm and approximately 7 mm.
